# EUROPÄISCHE PATENTANMELDUNG

(11) **EP 0 669 120 A1**
(43) Veröffentlichungstag der Anmeldung: **30.08.1995**
(21) Anmeldenummer: 95101058.6
(22) Anmeldetag: 26.01.1995
(51) Int. Cl.: A61F 13/15

(54) **Windel**

(30) Priorität: 28.01.1994 DE 9401412 U
(71) Anmelder: Gräther, Dieter, D-72818 Trochtelfingen (DE)
(72) Erfinder: Gräther, Dieter, D-72818 Trochtelfingen (DE)
(74) Vertreter: Möbus, Rudolf, Dipl.-Ing.

(57) **Zusammenfassung**

Eine Windel, die aus einem einstückigen Zuschnitt (10) aus einem waschbaren und wiederverwendbaren Material mit einfaltbaren Abschnitten (12, 13) gefertigt ist, wobei an den Umschlagstellen (16) der einfaltbaren Abschnitte (12, 13) jeweils eine Falthilfe (14) vorgesehen ist.

## Beschreibung

Die Erfindung betrifft eine Windel, vorzugsweise als Einlage in eine Windelhose.

Windeln dienen der Aufnahme von Ausscheidungen von Kleinkindern oder auch von inkontinenten Erwachsenen und wurden ursprünglich aus weichen, saugfähigen Tüchern gebildet, die zusammengelegt und um den Unterleib der betreffenden Person gewickelt und von einem darübergezogenen Höschen festgehalten wurden. In neuerer Zeit wurden diese Windeln, die meist aus Baumwolle gefertigt waren, durch aus Kunststoff bestehende Windelhöschen mit dicken, saugfähigen Einlagen ersetzt. Die Saugfähigkeit dieser Kunststoffwindeln ist sehr hoch, doch sind diese Windeln nicht wiederverwendbar. Durch den mehrmals täglich erforderlichen Windelwechsel entstehen damit beträchtliche Mengen an Abfall, dessen Entsorgung ein Problem darstellt. Außerdem kann insbesondere bei Kleinkindern ein zunehmendes Auftreten von durch Kunststoffwindeln hervorgerufene Allergien festgestellt werden. Aus gesundheits- und umweltpolitischen Gründen gewinnt daher seit kürzerem wieder die wiederverwendbare Windel aus einem natürlichen Material wie Baumwolle zunehmend an Attraktivität. Um eine ähnliche Saugfähigkeit wie bei Kunststoffwindeln zu erhalten, sind hierbei mittlerweile Windeln entwickelt worden, die aus mehreren, an den Seiten miteinander vernähten Tuchlagen gefertigt und in ihrem Zuschnitt der Körperform angepaßt sind. Diese Windeln sind sehr viel schneller anzulegen als die herkömmlichen Windeln, die aus einem rechteckigen Tuch gefaltet wurden. Sie haben jedoch den Nachteil, daß beim Waschen das aus mehreren Lagen bestehende Polster nur schwierig vollständig zu reinigen ist. Außerdem sind die Trockenzeiten dieser Windeln sehr lang. Zur Abhilfe dieses Problems ist eine Windeleinlage vorgeschlagen worden, die aus einer Grundlage und mehreren mit dieser teilweise vernähten Decklagen besteht. Die Decklage und die Grundlage können zum Waschen teilweise auseinandergefaltet werden, wodurch eine bessere Reinigung und auch ein schnelleres Trocknen ermöglicht wird. An den Nahtstellen jedoch bleibt der Nachteil einer Vielzahl von übereinanderliegenden Stofflagen bestehen. An diesen Stellen eingedrungener Schmutz kann nur sehr schwer wieder entfernt werden. Außerdem sind die Nahtstellen diejenigen Bereiche der Windel, die beim Trocknen am längsten feucht bleiben.

Der Erfindung liegt daher die Aufgabe zugrunde, eine Windel aus einem waschbaren und wiederverwendbaren Material zu schaffen, die rasch angelegt werden kann und dennoch leicht zu waschen und zu trocknen ist.

Die Aufgabe wird mit einer Windel der eingangs genannten Art erfindungsgemäß dadurch gelöst, daß sie aus einem einstückigen Zuschnitt aus einem waschbaren und wiederverwendbaren Material mit einfaltbaren Abschnitten gefertigt ist, wobei an den Umschlagstellen der einfaltbaren Abschnitte jeweils eine Falthilfe vorgesehen ist. Dabei können die Falthilfen vorteilhafterweise von Steppnähten gebildet sein. Eine solche Windel weist im ausgefalteten Zustand keine Abschnitte mit einer größeren Dicke auf. Insofern ist beim Waschen überall die gleiche Reinigungsqualität erzielbar, und die Windel trocknet anschließend vollständig gleichmäßig. Die Falthilfen geben vor, an welchen Stellen die einfaltbaren Abschnitte umzuschlagen sind, erleichtern das Falten und sorgen für einen korrekten Sitz der Windel. Zweckmäßigerweise können dabei die einfaltbaren Abschnitte links und rechts eines zentralen Winkelbereichs angeformt und von im wesentlichen rechteckförmiger Gestalt sein. Im zentralen Bereich der Windel muß die größte Flüssigkeitsmenge aufgenommen werden. Hier ist die Herstellung eines besonders saugfähigen, aus mehreren Lagen bestehenden Abschnitts erforderlich. Ein optimales Polster ergibt sich, wenn die einfaltbaren Abschnitte mindestens näherungsweise die gleiche Größe aufweisen und sich im eingefalteten Zustand nahezu vollständig überlappen. Der Saugbereich entspricht dann in seinen Abmessungen demjenigen einer Windel aus mehreren, an den Seiten miteinander vernähten Lagen, ohne jedoch deren Nachteile einer schlechten Waschbarkeit und langer Trockenzeiten aufzuweisen. Um ein beguemes Tragen der Windel zu ermöglichen, kann sie in gefaltetem Zustand der Körperform angepaßt sein. Außerdem kann auf der Innenseite der Windel eine Einlage aus Papiervlies befestigbar sein. Das Papiervlies dient dem Zurückhalten von Stuhlgang und verhindert dadurch eine zu starke Verschmutzung der Windel. Das Papiervlies wird nach Gebrauch der Windel weggeworfen, wobei das Papier als vollständig verrottbares Material kein Problem bei der Müllentsorgung darstellt. Vorteilhafterweise kann die Windel im wesentlichen aus Baumwolle als einem besonders körperverträglichen Material gefertigt sein. Außerdem kann sie mit Hilfe eines Klettverschlusses oder dergleichen an einer Windelhose befestigbar sein, wodurch sich ihr Gebrauch noch vereinfacht.

Bei einer vorteilhaften Ausgestaltung der Windel kann im Schrittbereich beidseitig ein außen gerafftes Ansatzstück als Auslaufschutz angeordnet sein, das sich beim Tragen eng an das Bein anlegt und somit ein Austreten von Flüssigkeit in diesem Bereich verhindert.

Nachfolgend wird ein bevorzugtes Ausführungsbeispiel einer erfindungsgemäßen Windel anhand der Zeichnung näher erläutert.

Im einzelnen zeigen:
Fig. 1 eine Draufsicht auf eine Windel;
Fig. 2 einen zentralen Querschnitt durch die Windel nach Fig. 1 in gefaltetem Zustand.

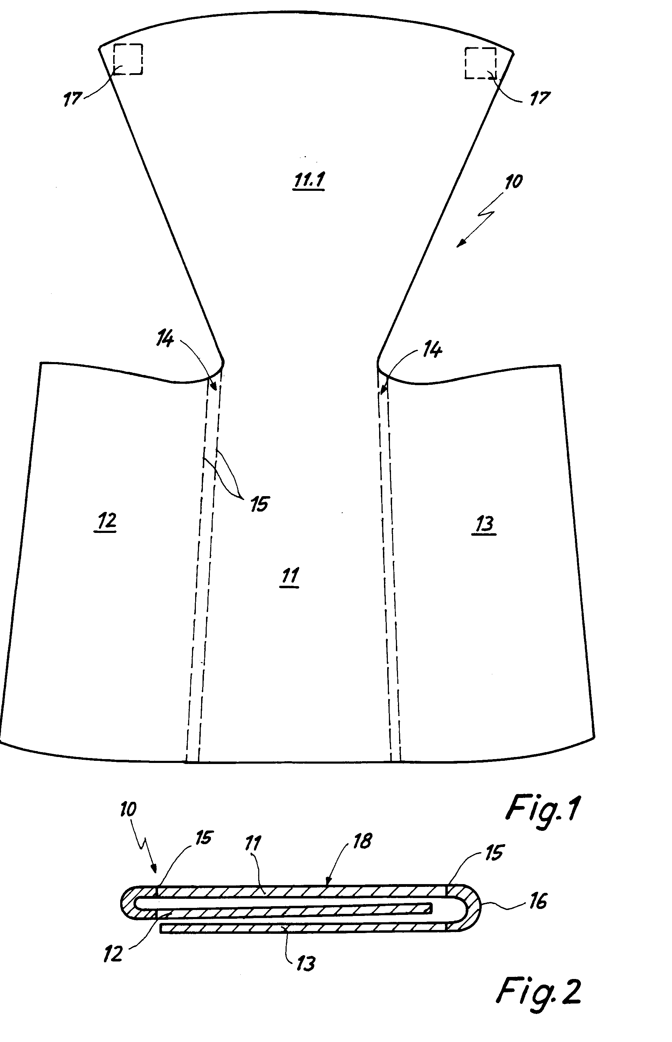

In Fig. 1 ist eine einteilige Windel 10 mit einem zentralen, im Schritt zu tragenden Bereich 11, der sich zur Körperrückseite hin zu einem um das Hinterteil des Kleinkindes zu legenden Teil 11.1. verbreitert. Links und rechts des zentralen Bereichs 11 sind zwei einfaltbare Abschnitte 12 und 13 vorgesehen, die in Form und Größe annähernd gleich sind und der rechteckigen Form und Größe des zentralen Bereichs 11 entsprechen. Die Abschnitte 12 und 13 werden, wie in Fig. 2 gezeigt, nach unten umgeschlagen, so daß sich ein Bereich 18 ergibt, in dem der zentrale Bereich 11, der Abschnitt 12 und der Abschnitt 13 übereinanderliegen und somit ein Polster hoher Saugfähigkeit bilden. Das Umschlagen der Abschnitte 12 und 13 wird jeweils durch eine Falthilfe 14, die von zwei Steppnähten 15 gebildet ist, erleichtert. Der zwischen den Steppnähten 15 liegende streifenförmige Bereich der Windel bildet nach dem Einschlagen der Abschnitte 12 und 13 die Umschlagkanten 16 (Fig. 2). In den äußeren Ecken des sich erweiternden Teils 11.1 der Windel 10 sind Befestigungsstellen 17, die jeweils mit feinen Häkchen zur Befestigung der Windel an einer Windelhose und zur Bildung eines Klettverschlusses mit derselben vorgesehen. Auf der Innenseite der Windel kann außerdem eine Einlage aus Papiervlies befestigt werden. Das Papiervlies kommt damit als innerste Lage der Windel direkt auf die Haut des Kleinkindes oder der inkontinenten Person zu liegen und verhindert ein Durchdringen von Stuhlgang auf die Windel 10.

Die in Fig. 1 dargestellte Form der Windel ist lediglich beispielhaft. Die Windel könnte auch anders geformt sein, beispielsweise im Bereich des Bauches ebenfalls einen sich erweiternden Abschnitt aufweisen. Auf der Außenseite der Windel 10 können in nicht dargestellter Weise Klettverschlüsse oder dergleichen zur Befestigung der Windel 10 an einer Windelhose vorgesehen sein.

## Patentansprüche

1. Windel, vorzugsweise als Einlage in eine Windelhose, dadurch gekennzeichnet, daß sie aus einem einstückigen Zuschnitt (10) aus einem waschbaren und wiederverwendbaren Material mit einfaltbaren Abschnitten (12, 13) gefertigt ist, wobei an den Umschlagstellen (16) der einfaltbaren Abschnitte jeweils eine Falthilfe (14) vorgesehen ist.

2. Windel nach Anspruch 1, dadurch gekennzeichnet, daß die Falthilfen (14) von Steppnähten (15) gebildet sind.

3. Windel nach Anspruch 1 oder 2, dadurch gekennzeichnet, daß die einfaltbaren Abschnitte (12, 13) links und rechts eines zentralen Windelbereichs (11) angeformt und von im wesentlichen rechteckförmiger Gestalt sind.

4. Windel nach Anspruch 3, dadurch gekennzeichnet, daß die einfaltbaren Abschnitte (12, 13) mindestens näherungsweise die gleiche Größe aufweisen und sich im eingefalteten Zustand nahezu vollständig überlappen.

5. Windel nach einem der Ansprüche 1 bis 4, dadurch gekennzeichnet, daß sie im gefalteten Zustand der Körperform angepaßt ist.

6. Windel nach einem der Ansprüche 1 bis 5, dadurch gekennzeichnet, daß auf der Innenseite der Windel (10) ein Papiervlies befestigbar ist.

7. Windel nach einem der Ansprüche 1 bis 6, dadurch gekennzeichnet, daß sie im wesentlichen aus Baumwolle gefertigt ist.

8. Windel nach einem der Ansprüche 1 bis 7, dadurch gekennzeichnet, daß sie mit Hilfe eines Klettverschlusses (17) oder dergleichen an einer Windelhose befestigbar ist.

9. Windel nach einem der Ansprüche 1 bis 8, dadurch gekennzeichnet, daß im Schrittbereich beidseitig ein außen gerafftes Ansatzstück als Auslaufschutz angeordnet ist.
